# EUROPEAN PATENT APPLICATION

(11) **EP 1 108 791 A1**
(43) Date of publication of application: **20.06.2001**
(21) Application number: 99125148.9
(22) Date of filing: 16.12.1999
(51) Int. Cl.: C12Q 1/68

(54) **Genetic variants of the human FSH receptor and the influence thereof on gametogenesis**

(71) Applicant: Gromoll, Jörg, 48147 Münster (DE)
(72) Inventor: Gromoll, Jörg, Dr., 48147 Münster (DE); Simoni, Manuela, Prof. Dr., 48147 Münster (DE); Nieschlag, Eberhard, Prof. Dr., 48167 Münster (DE); Behre, Hermann M., Dr., 48155 Münster (DE); Perez, Maritza, Dr. Institut für, Domagkstr 11, 48129 Münster (DE)
(74) Representative: Helbing, Jörg, Dr. Dipl.-Chem.

(57) **Abstract**

The present invention provides a method for determining the dosage of FSH in the treatment of infertility of women comprising the determination of the FSH receptor variant of the soman to be treated, a method for treating infertility in women which comprises said determination of the FSH receptor variant and a kit for performing said determination of the FSH receptor variant.

### Annex to the application documents-subsequently filed sequences listing

SEQUENCE LISTING

<110> GROMOLL, Jörg

<120> Genetic Variants of the Human FSH Receptor and the
      Influence Thereof on Gametogenesis

<130> 992882ep/JH/ml

<140> 99125145.9-2116
<141> 1999-12-16

<160> 19

<170> PatentIn Ver. 2.1


<210> 1
<211> 19
<212> DNA
<213> Artificial Sequence

<220>
<223> Description of Artificial Sequence: primer

<400> 1
ccttgtgcta atgtcctgg                                                19


<210> 2
<211> 20
<212> DNA
<213> Artificial Sequence

<220>
<223> Description of Artificial Sequence: primer

<400> 2
tgtagaagca ctgtcagctc                                               20


<210> 3
<211> 20
<212> DNA
<213> Artificial Sequence

<220>
<223> Description of Artificial Sequence: primer

<400> 3
gctatactgg atctgagatg                                               20


<210> 4
<211> 20
<212> DNA
<213> Artificial Sequence

<220>
<223> Description of Artificial Sequence: primer

<400> 4
ttgacatgac gtacactgag                                                    20

<210> 5
<211> 20
<212> DNA
<213> Artificial Sequence

<220>
<223> Description of Artificial Sequence: primer

<400> 5
ctgatctctg cattggaatc                                                    20

<210> 6
<211> 20
<212> DNA
<213> Artificial Sequence

<220>
<223> Description of Artificial Sequence: primer

<400> 6
agctggactg caaggtgcag                                                    20

<210> 7
<211> 20
<212> DNA
<213> Artificial Sequence

<220>
<223> Description of Artificial Sequence: primer

<400> 7
ccttgtgctc aatgtcctgg                                                    20

<210> 8
<211> 20
<212> DNA
<213> Artificial Sequence

<220>
<223> Description of Artificial Sequence: primer

<400> 8
ccatttctgc ctccctcaag                                                    20

<210> 9
<211> 20
<212> DNA
<213> Artificial Sequence

<220>
<223> Description of Artificial Sequence: primer

<400> 9
gagcaagtgt ggctgctatg                                                    20

<210> 10
<211> 20
<212> DNA
<213> Artificial Sequence

<220>
<223> Description of Artificial Sequence: primer

<400> 10
accacttcat tgcataagtc                                                    20


<210> 11
<211> 20
<212> DNA
<213> Artificial Sequence

<220>
<223> Description of Artificial Sequence: primer

<400> 11
caactgatgc aatgagcagc                                                    20


<210> 12
<211> 20
<212> DNA
<213> Artificial Sequence

<220>
<223> Description of Artificial Sequence: primer

<400> 12
atccagccca tcaccatgac                                                    20


<210> 13
<211> 20
<212> DNA
<213> Artificial Sequence

<220>
<223> Description of Artificial Sequence: primer

<400> 13
ggttccgcac tgtgaggtag                                                    20


<210> 14
<211> 20
<212> DNA
<213> Artificial Sequence

<220>
<223> Description of Artificial Sequence: primer

<400> 14
gctttggaca cagtgatgag                                                    20

<210> 15
<211> 20
<212> DNA
<213> Artificial Sequence

<220>
<223> Description of Artificial Sequence: primer

<400> 15
tggatgggtg ttgtggacag                                                    20


<210> 16
<211> 20
<212> DNA
<213> Artificial Sequence

<220>
<223> Description of Artificial Sequence: primer

<400> 16
tgtagaagca ctgtcagctc                                                    20


<210> 17
<211> 1243
<212> DNA
<213> Homo sapiens

<300>

<400> 17
tctagctctg agcttcatcc aatttgcaac aaatctattt taaggcaaga agttgattat 60
atgactcaga ctaggggtca gagatcctct ctggcagaag acaatgagtc cagctacagc 120
agaggatttg acatgacgta cactgagttt gactatgact tatgcaatga agtggttgac 180
gtgacctgct cccctaagcc agatgcattc aacccatgtg aagatatcat ggggtacaac 240
atcctcagag tcctgatatg gtttatcagc atcctggcca tcactgggaa catcatagtg 300
ctagtgatcc taactaccag ccaatataaa ctcacagtcc ccaggttcct tatgtgcaac 360
ctggcctttg ctgatctctg cattggaatc tacctgctgc tcattgcatc agttgatatc 420
cataccaaga gccaatatca caactatgcc attgactggc aaactggggc aggctgtgat 480
gctgctggct ttttcactgt ctttgccagt gagctgtcag tctacactcc gacagctatc 540
accttggaaa gatggcatac catcacgcat gccatgcagc tggactgcaa ggtgcagctc 600
cgccatgctg ccagtgtcat ggtgatgggc tggatttttg cttttgcagc tgccctcttt 660
cccatctttg gcatcagcag ctacatgaag gtgagcatct gcctgcccat ggatattgac 720
agccctttgt cacagctgta tgtcatgtcc ctccttgtgc tcaatgtcct ggcctttgtg 780
gtcatctgtg gctgctatat ccacatctac ctcacagtgc ggaaccccaa catcgtgtcc 840
tcctctagtg acaccaggtc gccaagcgca tggccatgct catcttcact gacttcctct 900
gcatggcacc catttctttc tttgccattt ctgcctccct caaggtgccc ctcatcactg 960
tgtccaaagc aaagattctg ctggttctgt ttcaccccat caactcctgt gccaacccct 1020
tcctctatgc catctttacc aaaaactttc gcagagattt cttcattctg ctgagcaagt 1080
gtggctgcta tgaaatgcaa gcccaaattt ataggacaga aacttcatcc actgtccaca 1140
acacccatcc aaggaatggc cactgctctt cagctcccag agtcaccagt ggttccacta 1200
acatacttgt ccctctaagt catttagccc aaaactaaaa cac                      1243


<210> 18
<211> 1450
<212> DNA
<213> Homo sapiens

<220>
<221> CDS

<222> (104)..(1333)

<400> 18
tctcaggaag aaactcatca tttcctaccc tgcacaaaga cagtgatgta ttgctatact 60

ggatctgaga tgttgattct atttcttttt gtatttttct agc tct gag ctt cat 115
Ser Glu Leu His
1

cca att tgc aac aaa tct att tta agg caa gaa gtt gat tat atg act 163
Pro Ile Cys Asn Lys Ser Ile Leu Arg Gln Glu Val Asp Tyr Met Thr
5                   10                  15                  20

cag act agg ggt cag aga tcc tct ctg gca gaa gac aat gag tcc agc 211
Gln Thr Arg Gly Gln Arg Ser Ser Leu Ala Glu Asp Asn Glu Ser Ser
                25                  30                  35

tac agc aga gga ttt gac atg acg tac act gag ttt gac tat gac tta 259
Tyr Ser Arg Gly Phe Asp Met Thr Tyr Thr Glu Phe Asp Tyr Asp Leu
            40                  45                  50

tgc aat gaa gtg gtt gac gtg acc tgc tcc cct aag cca gat gca ttc 307
Cys Asn Glu Val Val Asp Val Thr Cys Ser Pro Lys Pro Asp Ala Phe
        55                  60                  65

aac cca tgt gaa gat atc atg ggg tac aac atc ctc aga gtc ctg ata 355
Asn Pro Cys Glu Asp Ile Met Gly Tyr Asn Ile Leu Arg Val Leu Ile
    70                  75                  80

tgg ttt atc agc atc ctg gcc atc act ggg aac atc ata gtg cta gtg 403
Trp Phe Ile Ser Ile Leu Ala Ile Thr Gly Asn Ile Ile Val Leu Val
85                  90                  95                  100

atc cta act acc agc caa tat aaa ctc aca gtc ccc agg ttc ctt atg 451
Ile Leu Thr Thr Ser Gln Tyr Lys Leu Thr Val Pro Arg Phe Leu Met
                105                 110                 115

tgc aac ctg gcc ttt gct gat ctc tgc att gga atc tac ctg ctg ctc 499
Cys Asn Leu Ala Phe Ala Asp Leu Cys Ile Gly Ile Tyr Leu Leu Leu
            120                 125                 130

att gca tca gtt gat atc cat acc aag agc caa tat cac aac tat gcc 547
Ile Ala Ser Val Asp Ile His Thr Lys Ser Gln Tyr His Asn Tyr Ala
        135                 140                 145

att gac tgg caa act ggg gca ggc tgt gat gct gct ggc ttt ttc act 595
Ile Asp Trp Gln Thr Gly Ala Gly Cys Asp Ala Ala Gly Phe Phe Thr
    150                 155                 160

gtc ttt gcc agt gag ctg tca gtc tac act ctg aca gct atc acc ttg 643
Val Phe Ala Ser Glu Leu Ser Val Tyr Thr Leu Thr Ala Ile Thr Leu
165                 170                 175                 180

gaa aga tgg cat acc atc acg cat gcc atg cag ctg gac tgc aag gtg 691
Glu Arg Trp His Thr Ile Thr His Ala Met Gln Leu Asp Cys Lys Val
                185                 190                 195

cag ctc cgc cat gct gcc agt gtc atg gtg atg ggc tgg att ttt gct 739
Gln Leu Arg His Ala Ala Ser Val Met Val Met Gly Trp Ile Phe Ala
            200                 205                 210

ttt gca gct gcc ctc ttt ccc atc ttt ggc atc agc agc tac atg aag 787
Phe Ala Ala Ala Leu Phe Pro Ile Phe Gly Ile Ser Ser Tyr Met Lys
        215                 220                 225

gtg agc atc tgc ctg ccc atg gat att gac agc cct ttg tca cag ctg 835
Val Ser Ile Cys Leu Pro Met Asp Ile Asp Ser Pro Leu Ser Gln Leu
    230                 235                 240

tat gtc atg tcc ctc ctt gtg ctc aat gtc ctg gcc ttt gtg gtc atc 883
Tyr Val Met Ser Leu Leu Val Leu Asn Val Leu Ala Phe Val Val Ile
245                 250                 255                 260

tgt ggc tgc tat atc cac atc tac ctc aca gtg cgg aac ccc aac atc 931
Cys Gly Cys Tyr Ile His Ile Tyr Leu Thr Val Arg Asn Pro Asn Ile
                265                 270                 275

gtg tcc tcc tct agt gac acc agg atc gcc aag cgc atg gcc atg ctc 979
Val Ser Ser Ser Ser Asp Thr Arg Ile Ala Lys Arg Met Ala Met Leu
            280                 285                 290

atc ttc act gac ttc ctc tgc atg gca ccc att tct ttc ttt gcc att 1027
Ile Phe Thr Asp Phe Leu Cys Met Ala Pro Ile Ser Phe Phe Ala Ile
        295                 300                 305

tct gcc tcc ctc aag gtg ccc ctc atc act gtg tcc aaa gca aag att 1075
Ser Ala Ser Leu Lys Val Pro Leu Ile Thr Val Ser Lys Ala Lys Ile
    310                 315                 320

ctg ctg gtt ctg ttt cac ccc atc aac tcc tgt gcc aac ccc ttc ctc 1123
Leu Leu Val Leu Phe His Pro Ile Asn Ser Cys Ala Asn Pro Phe Leu
325                 330                 335                 340

tat gcc atc ttt acc aaa aac ttt cgc aga gat ttc ttc att ctg ctg 1171
Tyr Ala Ile Phe Thr Lys Asn Phe Arg Arg Asp Phe Phe Ile Leu Leu
                345                 350                 355

agc aag tgt ggc tgc tat gaa atg caa gcc caa att tat agg aca gaa 1219
Ser Lys Cys Gly Cys Tyr Glu Met Gln Ala Gln Ile Tyr Arg Thr Glu
            360                 365                 370

act tca tcc act gtc cac aac acc cat cca agg aat ggc cac tgc tct 1267
Thr Ser Ser Thr Val His Asn Thr His Pro Arg Asn Gly His Cys Ser
        375                 380                 385

tca gct ccc aga gtc acc agt ggt tcc act tac ata ctt gtc cct cta 1315
Ser Ala Pro Arg Val Thr Ser Gly Ser Thr Tyr Ile Leu Val Pro Leu
    390                 395                 400

agt cat tta gcc caa aac taaaacacaa tgtgaaaatg tatctgagta 1363
Ser His Leu Ala Gln Asn
405                 410

ttgaatgata aattcagtcc ttgcctttga agggtatgtc acaaggagct gacagtgctt 1423

ctacacattt tcatctaatt taatatt 1450

<210> 19
<211> 410
<212> PRT
<213> Homo sapiens

<400> 19
Ser Glu Leu His Pro Ile Cys Asn Lys Ser Ile Leu Arg Gln Glu Val
1               5                   10                  15

Asp Tyr Met Thr Gln Thr Arg Gly Gln Arg Ser Ser Leu Ala Glu Asp
            20                  25                  30

Asn Glu Ser Ser Tyr Ser Arg Gly Phe Asp Met Thr Tyr Thr Glu Phe
        35                  40                  45

Asp Tyr Asp Leu Cys Asn Glu Val Val Asp Val Thr Cys Ser Pro Lys
    50                  55                  60

Pro Asp Ala Phe Asn Pro Cys Glu Asp Ile Met Gly Tyr Asn Ile Leu
65                  70                  75                  80

Arg Val Leu Ile Trp Phe Ile Ser Ile Leu Ala Ile Thr Gly Asn Ile
                85                  90                  95

Ile Val Leu Val Ile Leu Thr Thr Ser Gln Tyr Lys Leu Thr Val Pro
            100                 105                 110

Arg Phe Leu Met Cys Asn Leu Ala Phe Ala Asp Leu Cys Ile Gly Ile
        115                 120                 125

Tyr Leu Leu Leu Ile Ala Ser Val Asp Ile His Thr Lys Ser Gln Tyr
    130                 135                 140

His Asn Tyr Ala Ile Asp Trp Gln Thr Gly Ala Gly Cys Asp Ala Ala
145                 150                 155                 160

Gly Phe Phe Thr Val Phe Ala Ser Glu Leu Ser Val Tyr Thr Leu Thr
                165                 170                 175

Ala Ile Thr Leu Glu Arg Trp His Thr Ile Thr His Ala Met Gln Leu
            180                 185                 190

Asp Cys Lys Val Gln Leu Arg His Ala Ala Ser Val Met Val Met Gly
        195                 200                 205

Trp Ile Phe Ala Phe Ala Ala Ala Leu Phe Pro Ile Phe Gly Ile Ser
    210                 215                 220

Ser Tyr Met Lys Val Ser Ile Cys Leu Pro Met Asp Ile Asp Ser Pro
225                 230                 235                 240

Leu Ser Gln Leu Tyr Val Met Ser Leu Leu Val Leu Asn Val Leu Ala
                245                 250                 255

Phe Val Val Ile Cys Gly Cys Tyr Ile His Ile Tyr Leu Thr Val Arg
            260                 265                 270

Asn Pro Asn Ile Val Ser Ser Ser Ser Asp Thr Arg Ile Ala Lys Arg
        275                 280                 285

Met Ala Met Leu Ile Phe Thr Asp Phe Leu Cys Met Ala Pro Ile Ser
    290                 295                 300

Phe Phe Ala Ile Ser Ala Ser Leu Lys Val Pro Leu Ile Thr Val Ser
305                 310                 315                 320

Lys Ala Lys Ile Leu Leu Val Leu Phe His Pro Ile Asn Ser Cys Ala
                325                 330                 335

Asn Pro Phe Leu Tyr Ala Ile Phe Thr Lys Asn Phe Arg Arg Asp Phe
            340                 345                 350

Phe Ile Leu Leu Ser Lys Cys Gly Cys Tyr Glu Met Gln Ala Gln Ile
        355                 360                 365

Tyr Arg Thr Glu Thr Ser Ser Thr Val His Asn Thr His Pro Arg Asn
    370                 375                 380

Gly His Cys Ser Ser Ala Pro Arg Val Thr Ser Gly Ser Thr Tyr Ile
385                 390                 395                 400

Leu Val Pro Leu Ser His Leu Ala Gln Asn
                405                 410

## Description

The present invention provides a method for determining the dosage of FSH in the treatment of infertility of women comprising the determination of the FSH receptor variant of the woman to be treated, a method for treating infertility in women which comprises said determination of the FSH receptor variant and a kit for performing said determination of the FSH receptor variant.

The success of controlled ovulation induction in the course of an assisted reproduction procedure depends on the administration of the hormone FSH. Unfortunately neither the patients's reaction towards the administration of FSH nor which hormone dose is necessary can be foreseen at the beginning of the treatment phase. Due to the lack of predictive parameters a high number of expensive ampoules of FSH is being used in IVF clinics for the induction of ovulation, a treatment regimen that bears the danger of overstimulation and its clinical consequences.

The follicle-stimulating hormone (FSH) is an essential factor for the maturation of germ cells (gametogenesis) in both men and women. FSH exerts its action via the FSH receptor which is specifically located in the granulosa cells in the ovary and in the Sertoli cells in the testis. Any perturbation of the interaction between FSH and its receptor leads to impairment of gametogenesis. Women with FSH receptor mutations show a clinical picture typical of primary amenorrhoea, men with FSH receptor mutation are generally subfertile. These observations underline the central role of FSH and its receptor for a normal physiological maturation of oocytes as well as spermatozoa (Nieschlag et al., Clin. Endocrinol. 51:139-146 (1999)).

The FSH receptor is present on the cell membrane and consists of an extracellular, a transmembrane and an intracellular domain. The FSH receptor gene is located on chromosome 2p21 and consists of 10 exons. Exons 1 to 9 encode for the extracellular domain while exon 10 encodes for the transmembrane and intracellular domain. The whole gene spans a region of over 54 kbp encoding a mature protein of 695 amino acids (Gromoll et al. Genomics 35, 308-311 (1996)).

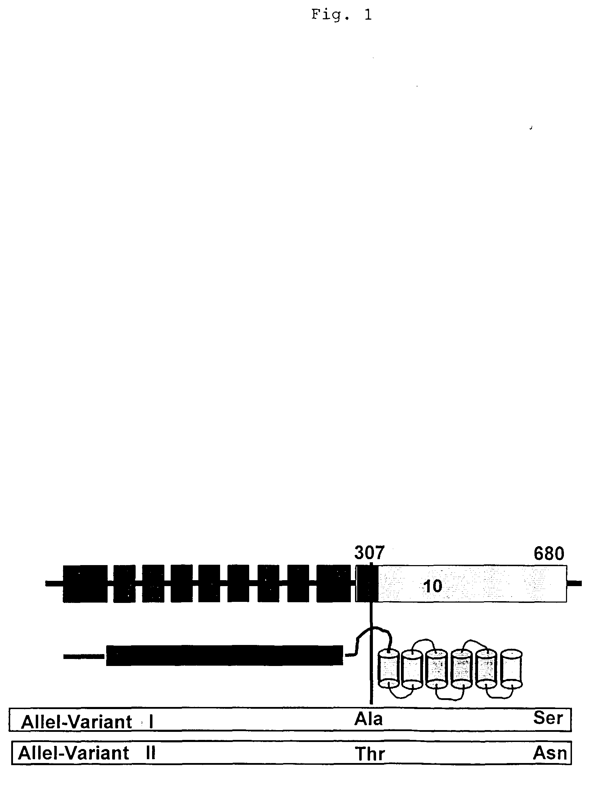

Our recent studies have shown that the FSH receptor exists in two genetic variants. Amino acid position 307 is either occupied by alanine or threonine and at position 680 either serine or asparagine is found. The amino acid position 307 is located in the extracellular domain while the amino acids at position 680 are part of the intracellular domain. We have shown that both positions are genetically linked with each other. Due to that two discrete FSH receptor variants can be usually found consisting of either threonine 307 and asparagine 680 or alanine 307 and serine 680 (Fig. 1). Both receptor variants are statistically equally distributed. So far functional studies could not show any significant differences as to hormone binding or signal transduction between the two receptor variants. It should be noted, however, that the model system used so far for functional studies is not sensitive enough to detect subtle differences in FSH-FSH receptor interaction and receptor activation. In a first clinical study comparing infertile men with fertile men no significant differences of receptor variant distribution could be detected (Simoni et al., J. Clin. Endocrinol. Metab. 84:751-755 (1999)).

In an additional study completed recently we have investigated normal women who were treated with FSH in the course of an assisted reproduction procedure. The administration of FSH leads to a controlled induction of ovulation which makes it possible to gain oocytes from the patient. The oocytes are then incubated with sperm in vitro and the nascent zygotes are re-implanted into the patient's uterus. The aim of the FSH treatment is to gain a sufficient number of oocytes capable of being fertilized. Clinical experience shows, however, that patients react differently towards the stimulation with FSH. While some patients need relatively low doses of FSH in order to produce a sufficient number of oocytes, other patients have to be stimulated with high doses of FSH to reach the same results in terms of a sufficient number of oocytes. Depending on the amount of FSH necessary the patients can be classified into good responders (low dose of FSH necessary) and bad responders (high dose of FSH necessary). The reasons for the difference in sensitivity towards stimulation with FSH are so far not known. The need for adjusting the FSH dose over the course of time during the stimulation phase in order to give rise to a sufficient number of oocytes without provoking an overstimulation represents a major problem in IVF treatment. The clinical picture of differences in the sensitivity towards FSH in patients undergoing assisted reproduction procedure was the starting point for our investigation in which we tested the hypothesis that different FSH receptor variants are responsible for the differences in FSH sensitivity.

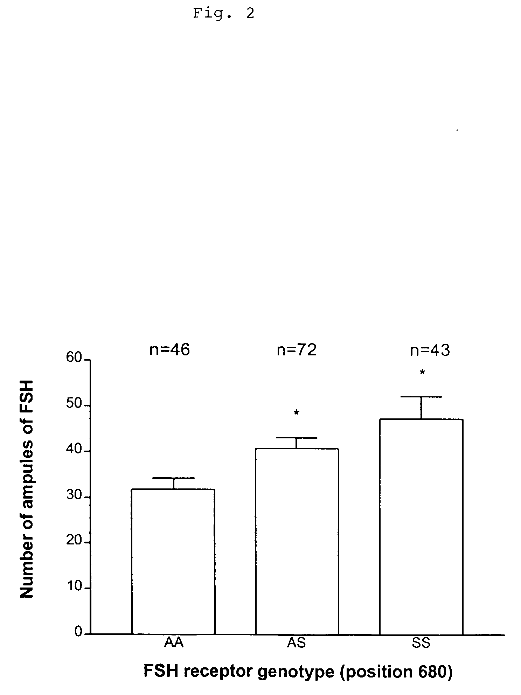

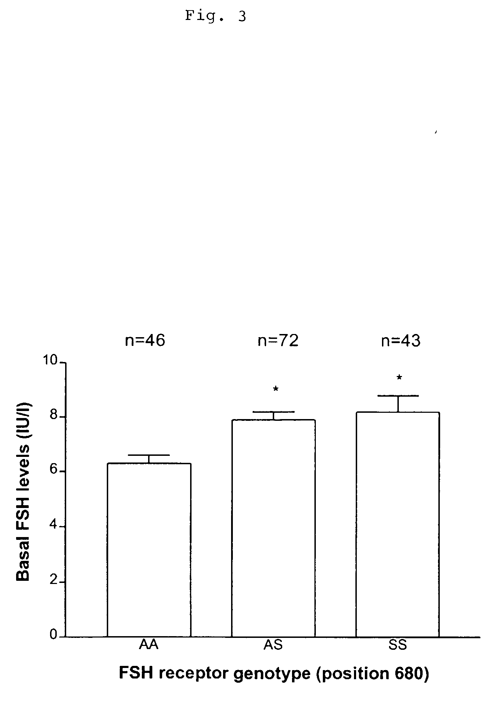

We have screened 160 patients from our IVF department and could show that patients bearing the homozygous FSH receptor variant alanine 307/serine 680 need significantly more FSH for the stimulation of oocyte maturation than the patients with the homozygous FSH receptor variant threonine 307/asparagine 680. It became further obvious that in patients with a heterozygous state of FSH receptor variants an intermediate dose of FSH was necessary for ovulation induction (Fig. 2). Moreover, the receptor variants seem to regulate the basal serum levels of FSH since patients bearing the homozygous FSH receptor variant alanine 307/serine 680 show a mean basal FSH level of 7.9 IU/I, while in patients with a heterozygous receptor variant and in patients homozygous for the FSH receptor variant threonine 307/asparagine 680 the mean basal FSH level is 7.0 IU/I and 6.3 IU/I, respectively (Fig. 3).

Thus, it was found that the response of patients towards the stimulation with FSH is depending on the allelic variant of the FSH receptor. In order to determine which particular variant of the FSH receptor is present in a given patient a simple analysis of DNA extracted from blood cells has to be conducted. The individual amount of FSH to be administered could be determined according to which FSH receptor variant the patient possesses. Due to these findings it can be expected that in the future the genetic analysis of the FSH receptor region determining the variant will play an important role for the planning of ovulation induction treatment. In other words, it was found that the FSH receptor variants determine differences in sensitivity towards FSH. This finding has a great impact on the FSH therapy in the course of an assisted reproduction procedure since it makes a predetermined and individually adjusted FSH stimulation protocol possible depending on the FSH receptor variant present in a given patient. The potential benefits of such an adjusted FSH stimulation therapy are profound. This approach will not only improve the clinical safety, by avoiding dangerous overstimulation with FSH, but will also help to reduce treatment costs significantly, since FSH is quite expensive.

Finally, FSH receptor variants may be associated with reduced fertility in men and women and that the analysis of such variants may have great impact on the treatment of reduced fertility with FSH.

The invention thus provides
(1) a method for determining the dosage of follicle-stimulating hormone (FSH) in the treatment of infertility of women comprising the determination of the FSH receptor variant of the woman to be treated;
(2) a method for treating infertility in women which comprises determining the FSH receptor variant in the woman to be treated as defined in (1) above; and
(3) a kit for performing the determination of the FSH receptor variant in a woman as defined in (1) and (2) above.

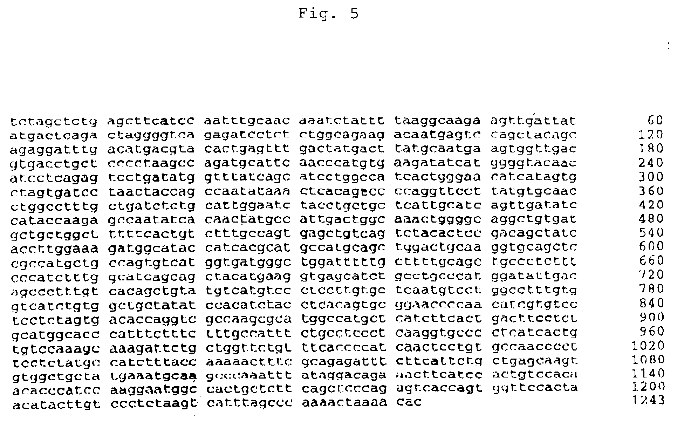

The attached Figures 1 to 6 depict the following:
Fig. 1 shows the two FSH receptor variants.
Fig. 2 shows the number of FSH ampoules (75 IU/ampoule) required to achieve ovulation induction and oocyte retrieval in normoovulatory women in an assisted reproduction programme.
   (data: mean ± SEM)
   * p < 0.05 vs A/A (Kruskall Wallis test)
Fig. 3 shows the basal FSH levels (day 3) in normoovulatory women grouped according to the FSH receptor genotype.
   (data: mean ± SEM)
   *p < 0.05 vs A/A (Kruskall Wallis test)
Fig. 4 shows the restriction fragment length polymorphism of Asn 680 Ser of the FSH receptor.
Fig. 5 shows the DNA sequence of exon 10 of the FSH receptor gene (EMBL accession No. X91747).
Fig. 6 shows exon 10 of the FSH receptor. Suitable primers are underlined. The reverse primers A₂-G₂ are complementary to the respective underlined sequences in this figure.

The method (1) of the invention is hereinafter described in more detail. First of all, the determination of the FSH receptor variant is preferably performed *in vitro.*

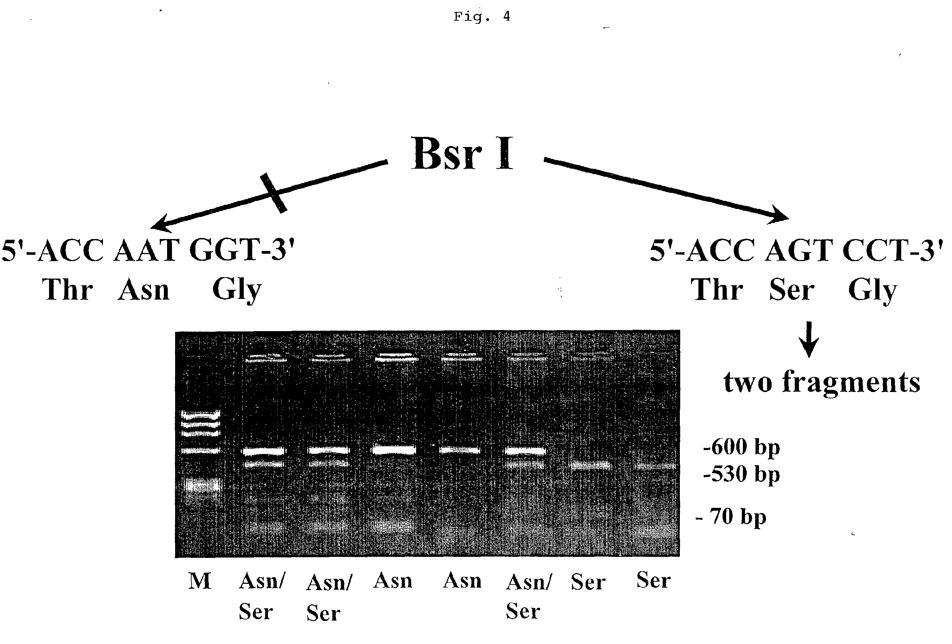

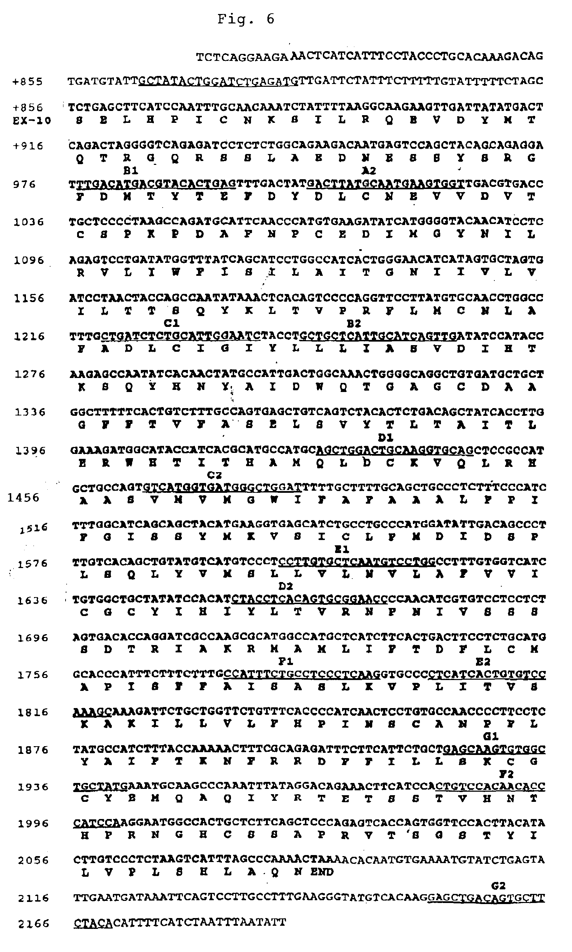

Secondly, since the polymorphic sites are genetically coupled in the FSH receptor variants, the sequence analysis of one variant site is sufficient. It is preferred that the analysis is performed by restriction fragment length polymorphism (RFLP). The first step is the isolation of genomic DNA from a patient's blood sample and the amplification of a specific part of the FSH receptor by PCR. The amplicon obtained in the PCR is cut with a restriction enzyme which specifically recognizes the amino acid sequence at postion 680, e.g. Bsr I. A complete restriction of the amplicon indicates the presence of a homozygous serine, in the case of an incomplete restriction a heterozygous receptor status is present and no restriction of the amplicon indicates a homozygous asparagine (Fig. 4). Suitable primers for the PCR reaction are shown in Fig. 6.

The detection of a single nucleotide change leading to an amino acid exchange is ideally suited for the development of a specific kit which would make the detection of the FSH receptor variants easy and fast. Such a kit would ideally be used for the screening of patients prior to a FSH therapy.

In the FSH therapy and after determination of the FSH receptor the woman may be given a high dosage of FSH (42-48 ampoules FSH within a treatment period of 14 days) or a low dosage of FSH (30-35 ampoules FSH per 14 days).

The invention is further illustrated by the following non-limitative example.

### Example: Determination of the restriction fragment length polymorphism (Asn680Ser, hFSH receptor)

### A. PCR: Amplification with primers E₁ and G₂ (Exon 10 of the FSH receptor)

For each sample is pipetted:
36 µl autoclaved distilled water
5 µl Thermo-Buffer 10x (Promega)
3 µl MgCl₂ (25 mM, Promega)
2.5 µl dNTP-solution (1mM, Pharmacia)
1 µl Primer E₁ (0.1 µg/µl; 5'-CCTTGTGCTAATGTCCTGG)
1 µl Primer G₂ (0.1 µg/µl; 5'-TGTAGAAGCACTGTCAGCTC)
0.5 µl Taq DNA polymerase (5000 lU/ml, Promega)
1 µl DNA (DNA extracted from 5-10 ml and dissolved in 50 µl distilled water)

| PCR program: | | |
|---|---|---|
| 94°C | 4 min | 1 cycle |
| | | |
| 94°C | 1 min | |
| 58°C | 30 s | 35 cycles |
| | | |
| 72°C | 50 s | |
| 72°C | 10 min | 1 cycle |
| 30°C | 30 min | |

The PCR product is checked on a 2% TAE agarose gel. The size of the desired band is 580 kbp.
Subsequently a phenol-chloroform cleaning is performed twice, the resulting DNA is precipitated with 0.5 sample volumes 7.5 M ammonium acetate and 2.5 volumes absolute ethanol, washed with 70% ethanol and air-dried. Finally, the DNA is redissolved in 17 µl water.

### B. Digestion:

2 µl buffer NEB 3 10x (Biolabs) and
1 µl Bsr I (Biolabs) are added to the sample, the resulting mixture is overlayed with 2 drops of mineral oil and digested for 1.5 hours at 65°C. The digestion is checked on a 2-2.5% TAE agarose gel.

The enzyme Bsr I has a restriction site for TGACC. If the FSH receptor contains the amino acid serin at position 680 of the 5^{th} transmembrane domain, Bsr I cuts the PCR product in two bands (443 and 136 bp). The enzyme cannot digest the PCR product if the amino acid at position 680 is asparagin. The single band on the gel has a size of 579 bp (see Fig. 4).

### C. Results:

- One band size 579 bp →: asparagin, homozygous
- Two band sizes 443 and 136 bp →: serin, homozygous
- Three band sizes 579, 443 and 136 bp →: asparagin/serin heterozygous

## Claims

1. Method for determining the dosage of follicle-stimulating hormone (FSH) in the treatment of infertility of women comprising the determination of the FSH receptor variant of the woman to be treated.

2. The method of claim 1, wherein the determination of the FSH receptor variant comprises the steps:
(a) isolating genomic DNA from a blood sample of the woman to be treated, and
(b) determining whether the isolated DNA codes for the FSH-receptor variant Ala 307/Ser 680 or Thr 307/Asn 680.

3. The method of claim 2, wherein the determination of the FSH-receptor variant of step (b) is performed by
(b1) partial amplification of the FSH receptor DNA by use of a pair of primers flanking the variant region(s) of the FSH receptor DNA coding for the amino acids 307 and/or 680 of the FSH receptor protein,
(b2) digesting the amplified DNA with a restriction enzyme digesting only the DNA of one of the FSH receptor variants,
(b3) determining the FSH-receptor variant by restriction fragment lengthpolymorphism.

4. The method of claim 3, wherein the length of the primers is 12 to 30 nucleotides, preferably 17 to 25 nucleotides, and the distance to the nucleotides coding for the amino acid in positions 307 or 680 are 20 to 1500 bp, preferably 100 to 1000 bp.

5. The method of claim 3 or 4, wherein the primers are flanking the DNA sequence of the amino acid in position 680 of the FSH receptor protein and the restriction enzyme is Bsr I.

6. The method of claims 3 to 5, wherein the pair of primers comprises an upstream primer selected from 
A₁: 5'-GCTATACTGGATCTGAGATG
B₁: 5'-TTGACATGACGTACACTGAG
C₁: 5'-CTGATCTCTGCATTGGAATC
D₁: 5'-AGCTGGACTGCAAGGTGCAG
E₁: 5'-CCTTGTGCTCAATGTCCTGG
F₁: 5'-CCATTTCTGCCTCCCTCAAG
G₁: 5'-GAGCAAGTGTGGCTGCTATG,
 and a reverse primer selected from 
A2: 5'-ACCACTTCATTGCATAAGTC
B2: 5'-CAACTGATGCAATGAGCAGC
C2: 5'-ATCCAGCCCATCACCATGAC
D2: 5'-GGTTCCGCACTGTGAGGTAG
E2: 5'-GCTTTGGACACAGTGATGAG
F2: 5'-TGGATGGGTGTTGTGGACAG
G2: 5'-TGTAGAAGCACTGTCAGCTC,
 preferably the pair of primers is E₁ and G₂ as defined above.

7. A method for treating infertility in women which comprises determining the FSH receptor variant in the woman to be treated as defined in claims 1 to 6.

8. The method of claim 7, further comprising administering the woman a suitable amount of FSH.

9. A kit for performing the determination of the FSH receptor variant in a woman as defined in claims 1 to 8.

10. The kit of claim 9 comprising a pair of primers as defined in claims 3-6, Taq polymerase and a restriction enzyme.
